(19) Européisches Patentamt / European Patent Office / Office européen des brevets

(11) **EP 4 618 005 A1**

(12) **EUROPEAN PATENT APPLICATION**

(43) Date of publication:
**17.09.2025 Bulletin 2025/38**

(21) Application number: **24162977.3**

(22) Date of filing: **12.03.2024**

(51) International Patent Classification (IPC):
**G06T 7/00** $^{(2017.01)}$      **G06T 7/20** $^{(2017.01)}$
**A61B 3/10** $^{(2006.01)}$      **A61B 3/12** $^{(2006.01)}$

(52) Cooperative Patent Classification (CPC):
**G06T 7/20; G06T 7/0012;** A61B 3/102; A61B 3/12;
G06T 7/579; G06T 2207/10101; G06T 2207/20216;
G06T 2207/30041

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Optos plc
Dunfermline, Scotland KY11 8GR (GB)**

(72) Inventors:
• **PRECIADO, Miguel**
  **Dunfermline, Scotland, KY11 8GR (GB)**
• **RYCROFT, Ewan**
  **Dunfermline, Scotland, KY11 8GR (GB)**
• **McCOOL, Paul**
  **Dunfermline, Scotland, KY11 8GR (GB)**
• **NORMAND, Margaret**
  **Dunfermline, Scotland, KY11 8GR (GB)**

(74) Representative: **Hoffmann Eitle
Patent- und Rechtsanwälte PartmbB
Arabellastraße 30
81925 München (DE)**

(54) **PROCESSING TECHNIQUES FOR OPTORETINOGRAPHY**

(57)      A computer-implemented method of processing each of a plurality of sets of OCT images in a sequence of OCT images of a portion of a retina to generate a respective indication of a tissue velocity at a position along an axial direction in the OCT images, by performing, for each set: calculating a respective comparison value based on an image quality or similarity of a first OCT image and a second OCT image in the set; using phase information in the first OCT and second OCT image in a calculation of the respective indication of tissue velocity at the position if the comparison value is equal to or greater than a threshold; and omitting the phase information from the calculation if the comparison value is smaller than the threshold.

Fig. 3

EP 4 618 005 A1

**Description**

[Field]

Example aspects herein generally relate to the field of optical coherence tomography (OCT) and, in particular, to techniques for processing OCT data generated by Fourier-domain OCT imaging systems to generate optoretinography (ORG) data indicative of a physiological response of a retina of an eye of a subject to an optical stimulus.

[Background]

Optical coherence tomography (OCT) is an imaging technique based on low-coherence interferometry, which is widely used to acquire high-resolution two- and three-dimensional images of optical scattering media, such as biological tissue.

OCT imaging systems can be classified as being time-domain OCT (TD-OCT) or Fourier-domain OCT (FD-OCT) (also referred to as frequency-domain OCT), depending on how depth ranging is achieved. In TD-OCT, an optical path length of a reference arm of the imaging system's interferometer is varied in time during the acquisition of a reflectivity profile of the scattering medium being imaged by the OCT imaging system (referred to herein as the "imaging target"), the reflectivity profile being commonly referred to as a "depth scan" or "axial scan" ("A-scan"). In FD-OCT, a spectral interferogram resulting from an interference between light in the reference arm and light in the sample arm of the interferometer at each A-scan location is Fourier transformed to simultaneously acquire all points along the depth of the A-scan, without requiring any variation in the optical path length of the reference arm. FD-OCT can allow much faster imaging than scanning of the sample arm mirror in the interferometer, as all the back-reflections from the sample are measured simultaneously. Two common types of FD-OCT are spectral-domain OCT (SD-OCT) and swept-source OCT (SS-OCT). In SD-OCT, a broadband light source delivers many wavelengths to the imaging target, and all wavelengths are measured simultaneously using a spectrometer as the detector. In SS-OCT (also referred to as time-encoded frequency-domain OCT), the light source is swept through a range of wavelengths, and the temporal output of the detector is converted to spectral interference.

OCT imaging systems can also be classified as being point-scan (also known as "point detection" or "scanning point"), line-scan or full-field, depending on how the imaging system is configured to acquire OCT data at locations on the imaging target. A point-scan OCT imaging system acquires OCT data by scanning a focused sample beam across the surface of the imaging target, typically along a single line (which may be straight, or alternatively curved so as to define a circle or a spiral, for example) or along a set of (usually substantially parallel) lines on the surface of the imaging target, and acquiring an axial depth profile (A-scan) for each of a plurality of points along the line(s), one single point at a time, to build up OCT data comprising a one- or two-dimensional array of A-scans representing a two-dimensional (i.e. a B-scan) or three-dimensional (i.e. a C-scan or volumetric scan) reflectance profile of the sample.

A line-scan OCT imaging system acquires OCT data by scanning a focused line of light across the surface of the imaging target. Measured reflectance from the imaging target is used to generate OCT data comprising a two-dimensional reflectance profile (i.e. a B-scan) of the sample. By scanning the focused line of light across a plurality of locations on the imaging target, OCT data comprising a three-dimensional reflectance profile (i.e. a C-scan or volumetric scan) of the sample can be obtained. Typically, the focused line of light is straight and is scanned in a direction perpendicular to it, although in some instances it may be curved with the scanning direction adjusted accordingly. A full-field OCT imaging system acquires OCT data by projecting a beam of light onto the imaging target to acquire OCT data comprising a three-dimensional reflectance profile (i.e. a C-scan or volumetric scan) of the sample.

OCT imaging systems can also be classified as being phase-resolved, where both the intensity and phase of the light reflected from the imaging target are measured as a function of axial depth. Modern FD-OCT imaging systems often have a degree of phase stability that allows them to function as phase-resolved OCT imaging systems.

Optoretinography (ORG) generally refers to the detection of a physiological response of a retina of an eye to an optical stimulus (i.e. light-induced functional activity of the retina). ORG techniques include the non-invasive optical imaging of this physiological response of the retina. For example, OCT imaging systems can be used to image retinal neurons exhibiting a change in dimension (size) in response to excitation by the optical stimulus. These changes in dimension (typically changes in length of the photoreceptor outer segment (OS) in the retina, which is the difference in depth of the inner-outer segment (IS/OS) junction and the cone outer segment tip (COST) of the cone photoreceptors) lead to changes in the phase of the light waves returned from the eye that are big enough to be detectable by phase-resolved OCT imaging systems. The phase is very sensitive to movement in the tissue, and many phase-resolved OCT imaging systems are able to resolve displacements smaller than 10 nm, which may be much smaller than the axial resolution of the system or the wavelength of the light used in imaging.

[Summary]

There is provided, in accordance with a first example aspect herein, a computer-implemented method of processing each set of OCT images comprising a first OCT image and a second OCT image of a plurality of different sets of OCT images in a sequence of OCT images of a common portion of a retina of an eye acquired by a Fourier-domain OCT imaging system to generate a respective indication of a tissue velocity at a position along an axial direction in the OCT images. The method comprises performing, for each set of the sets of OCT images, processes of: calculating a respective comparison value being one of (i) a value of an image quality metric calculated based on at least one of the first OCT image in the set and the second OCT image in the set, and (ii) a value of an image similarity metric that provides a measure of a degree of similarity between images, the value of the image similarity metric being calculated based on the first OCT image in the set and the second OCT image in the set; calculating the respective indication of tissue velocity at the position along the axial direction using a phase value at the position along the axial direction in an A-scan of the first OCT image in the set, and a phase value at the position along the axial direction in a corresponding A-scan of the second OCT image in the set; comparing the comparison value with a threshold to determine whether the comparison value is equal to or greater than the threshold; in case the comparison value is determined to be equal to or greater than the threshold, retaining the respective indication of tissue velocity at the position along the axial direction; and in case the comparison value is determined not to be equal to or greater than the threshold, discarding the respective indication of tissue velocity at the position along the axial direction.

There is provided, in accordance with a second example aspect herein, a computer-implemented method of processing each set of OCT images comprising a first OCT image and a second OCT image of a plurality of different sets of OCT images in a sequence of OCT images of a common portion of a retina of an eye acquired by a Fourier-domain OCT imaging system to generate a respective indication of a tissue velocity (v) at a position along an axial direction in the OCT images. The method comprises performing, for each set of the sets of OCT images, processes of: calculating a respective comparison value being one of (i) a value of an image quality metric calculated based on at least one of the first OCT image in the set and the second OCT image in the set, and (ii) a value of an image similarity metric that provides a measure of a degree of similarity between images, the value of the image similarity metric being calculated based on the first OCT image in the set and the second OCT image in the set; comparing the comparison value with a threshold to determine whether the comparison value is equal to or greater than the threshold; in a case the comparison value is determined to be equal to or greater than the threshold, using a phase value at the position along the axial direction in an A-scan of the first OCT image in the set, and a phase value at the position along the axial direction in a corresponding A-scan of the second OCT image in the set, in a calculation of the respective indication of tissue velocity at the position along the axial direction; and in case the comparison value is determined not to be equal to or greater than the threshold, omitting a phase value at the position along the axial direction in an A-scan of the first OCT image in the set, and a phase value at the position along the axial direction in a corresponding A-scan of the second OCT image in the set, in the calculation of the respective indication of tissue velocity at the position along the axial direction.

In the methods set out above, the comparison value may be a maximum value of a calculated cross-correlation between the first OCT image and the second OCT image. Other similarity measures that could alternatively be used are sum of squared differences, mutual information, normalised mutual information, and Kullback Leibler distance, for example.

The computer-implemented method of the first example aspect or the second example aspect may further comprise generating a concatenation of the generated indications of tissue velocity, such that the concatenation is indicative of how the tissue velocity at the position along the axial direction changes over time, and integrating the concatenation to generate data indicating an optical path length variation over time at the position along the axial direction. In the case where the comparison value is determined not to be equal to or greater than the threshold, the respective indication of tissue velocity at the position along the axial direction is set to indicate zero velocity, such that the generated data comprises one or more sets of equal consecutive values, and the method may further comprise smoothing the generated data by replacing one or more values in a set of the one or more sets of equal consecutive values with one or more estimated values calculated based on neighbouring values that neighbour the set of equal consecutive values in the generated data.

Furthermore, in any of the foregoing, the computer-implemented method may further comprise processing the phase value at the position along the axial direction in the A-scan of the first OCT image in the set, and the phase value at the position along the axial direction in the corresponding A-scan of the second OCT image in the set, before the calculation of the respective indication of tissue velocity at the position along the axial direction, to compensate for a bulk motion of the common portion of the retina during acquisition of the sequence of OCT images by the Fourier-domain OCT imaging system.

There is provided, in accordance with a third example aspect herein, a computer program comprising computer-readable instructions which, when executed by a processor, cause the processor to perform the method of the first example aspect or the second example, or any of their variants set out above. The computer program may be stored on a non-transitory computer-readable storage medium (such as a computer hard disk or a CD, for example) or carried by a computer-readable signal.

There is provided, in accordance with a fourth example aspect herein, a data processing apparatus comprising a processor and a memory storing computer-readable instructions which, when executed by the processor, cause the processor to perform the method of the first example aspect or the second example, or any of their variants set out above.

There is provided, in accordance with a fifth example aspect herein, a data processing apparatus arranged to process each set of optical coherence tomography, OCT, images comprising a first OCT image and a second OCT image of a plurality of different sets of OCT images in a sequence of OCT images of a common portion of a retina of an eye (20) acquired by a Fourier-domain OCT imaging system to generate a respective indication of a tissue velocity at a position along an axial direction in the OCT images. The data processing apparatus is arranged to perform, for each set of the sets of OCT images, processes of: calculating a respective comparison value being one of (i) a value of an image quality metric calculated based on at least one of the first OCT image in the set and the second OCT image in the set, and (ii) a value of an image similarity metric that provides a measure of a degree of similarity between images, the value of the image similarity metric being calculated based on the first OCT image in the set and the second OCT image in the set; calculating the respective indication of tissue velocity at the position along the axial direction using a phase value at the position along the axial direction in an A-scan of the first OCT image in the set, and a phase value at the position along the axial direction in a corresponding A-scan of the second OCT image in the set; comparing the comparison value with a threshold to determine whether the comparison value is equal to or greater than the threshold; in case the comparison value is determined to be equal to or greater than the threshold, retaining the respective indication of tissue velocity at the position along the axial direction; and in case the comparison value is determined not to be equal to or greater than the threshold, discarding the respective indication of tissue velocity at the position along the axial direction.

There is provided, in accordance with a sixth example aspect herein, a data processing apparatus arranged to process each set of optical coherence tomography, OCT, images comprising a first OCT image and a second OCT image of a plurality of different sets of OCT images in a sequence of OCT images of a common portion of a retina of an eye acquired by a Fourier-domain OCT imaging system to generate a respective indication of a tissue velocity at a position along an axial direction in the OCT images. The data processing apparatus is arranged to perform, for each set of the sets of OCT images, processes of: calculating a respective comparison value being one of (i) a value of an image quality metric calculated based on at least one of the first OCT image in the set and the second OCT image in the set, and (ii) a value of an image similarity metric that provides a measure of a degree of similarity between images, the value of the image similarity metric being calculated based on the first OCT image in the set and the second OCT image in the set; comparing the comparison value with a threshold to determine whether the comparison value is equal to or greater than the threshold; in a case the comparison value is determined to be equal to or greater than the threshold, using a phase value at the position along the axial direction in an A-scan of the first OCT image in the set, and a phase value at the position along the axial direction in a corresponding A-scan of the second OCT image in the set, in a calculation of the respective indication of tissue velocity at the position along the axial direction; and in case the comparison value is determined not to be equal to or greater than the threshold, omitting a phase value at the position along the axial direction in an A-scan of the first OCT image in the set, and a phase value at the position along the axial direction in a corresponding A-scan of the second OCT image in the set, in the calculation of the respective indication of tissue velocity at the position along the axial direction.

The comparison value may be a maximum value of a calculated cross-correlation between the first OCT image and the second OCT image. Additionally or alternatively, the data processing apparatus may be further arranged to: generate a concatenation of the generated indications of tissue velocity such that the concatenation is indicative of how the tissue velocity at the position along the axial direction changes over time; and integrate the concatenation to generate data indicating an optical path length variation over time at the position along the axial direction. In the case where the comparison value is determined not to be equal to or greater than the threshold, the data processing apparatus may be arranged to set the respective indication of tissue velocity at the position along the axial direction to indicate zero velocity, the generated data may comprise one or more sets of equal consecutive values, and the data processing apparatus may be further arranged to smooth the generated data by replacing one or more values in a set of the one or more sets of equal consecutive values with one or more estimated values calculated based on neighbouring values that neighbour the set of equal consecutive values in the generated data.

The data processing apparatus may be further arranged to process the phase value at the position along the axial direction in the A-scan of the first OCT image in the set, and the phase value at the position along the axial direction in the corresponding A-scan of the second OCT image in the set, before the calculation of the respective indication of tissue velocity at the position along the axial direction, to compensate for a bulk motion of the common portion of the retina during acquisition of the sequence of OCT images by the Fourier-domain OCT imaging system.

There is provided, in accordance with a seventh example aspect herein, a Fourier-domain OCT imaging system comprising a data processing apparatus as set out in any of the foregoing.

[Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.

Figure 1 is a schematic illustration of a system comprising a Fourier-domain OCT imaging system 30 and a data processing apparatus 100 according to example embodiments herein.

Figure 2 is a schematic illustration of an example implementation of the data processing apparatus 100 in programmable signal processing hardware.

Figure 3 is a flow diagram illustrating a method according to a first example embodiment herein, by which OCT images are processed to generate indications of tissue velocity.

Figure 4A shows a first pair of B-scans that are highly correlated.

Figure 4B shows a calculated cross-correlation for the B-scans in Figure 4A.

Figure 5A shows a second pair of B-scans that are not highly correlated.

Figure 5B shows a calculated cross-correlation for the B-scans in Figure 5A.

Figure 6A illustrates a sinusoidal bulk oscillation added to oscillatory movements of two model retinal layers.

Figure 6B shows the effect of removing the bulk motion on the variation with time of the phase angle of light reflected from the two model retinal layers.

Figure 7 shows an example of a concatenation of the velocity profiles that may be generated by the data processing apparatus 100.

Figure 8 shows a plot of changes in optical path length $\Delta OPL$ over time, which have been calculated by a data processing apparatus of an example embodiment for a selected position in the velocity profiles of a concatenation of velocity profiles.

Figure 9 shows a plot of an example ORG signal generated by a data processing apparatus of an example embodiment herein.

Figure 10 shows a result of smoothing the ORG signal in Figure 9.

Figure 11 is a flow diagram illustrating optional processes that may be performed by the data processing apparatus 100 of the embodiments described herein.

Figure 12 is a flow diagram illustrating a method according to a second example embodiment herein, by which OCT images are processed to generate indications of tissue velocity.

[Detailed Description of Example Embodiments]

A core part of the process of generating ORG data from a set of repeat B-scans (or other OCT images) that have been acquired by an FD-OCT imaging system following the application of a light stimulus is to process each subset of the B-scans to generate a respective indication of a tissue velocity at one or more positions along an axial direction in the B-scans. The present inventors have recognised that some of the indications of tissue velocity calculated using conventional ORG processing techniques may be unreliable and adversely affect the ORG data or other final processing result. The inventors have furthermore devised schemes for recognising source B-scans (or other OCT images) that give rise to such unreliable indications, and for preventing any unreliable indications of tissue velocity from entering the processing pipeline, in order to improve the ORG data or other final processing result. Example embodiments will now be described in detail, with reference to the accompanying drawings.

[First Example Embodiment]

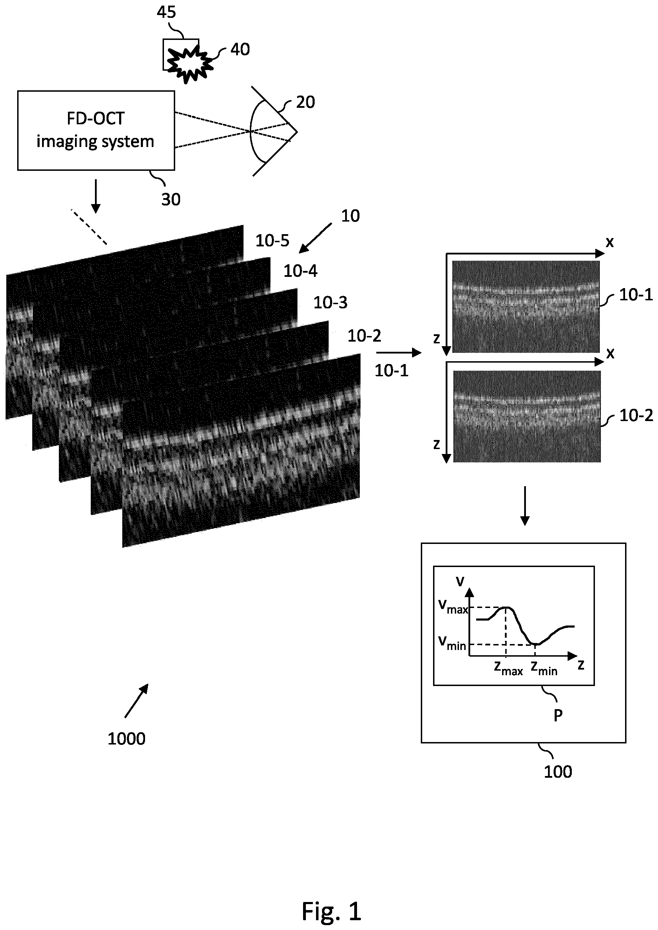

Figure 1 is a schematic illustration of a data processing apparatus 100 according to a first example embodiment. The data processing apparatus 100 is arranged to process complex OCT data generated by a phase-resolved Fourier-domain OCT (FD-OCT) imaging system. More specifically, the data processing apparatus 100 is arranged to process each set of OCT images comprising a first OCT image 10-1 and a second OCT image 10-2 of a plurality of different sets of OCT images in a sequence of OCT images 10 of a common portion of a retina of an eye 20 captured by a Fourier-domain OCT imaging system 30, to generate a respective indication of a tissue velocity v at one or more positions along an axial direction z in the OCT images 10 (i.e. the direction in any of the OCT images (e.g. B-scans or C-scans) along which the elements of a component A-scan of the OCT image are arrayed).

The FD-OCT imaging system 30 may, as in the present example embodiment, be a swept-source OCT (SS-OCT) system. However, the FD-OCT imaging system 30 need not be provided in this form and may, for example, take the alternative form of a spectral-domain OCT (SD-OCT). More generally, an example embodiment may be provided as any form of phase-resolved FD-OCT imaging system that can generate complex OCT data, i.e. Fourier transforms of respective spectral interferograms (interference spectra) representing complex A-scan information obtained for each scan location at which an OCT measurement is made during the scan. Such complex OCT data encodes phase information from acquired OCT measurements that can be used by the data processing apparatus 100 as described herein to calculate tissue velocities in the common portion of the retina.

The FD-OCT imaging system 30 may include well-known components, including a scanning system, a light detector, OCT data processing hardware, and a light beam generator (not shown). The scanning system may be arranged to perform a one- and/or two-dimensional point-scan of a light beam across the retina, and collect light which has been scattered by the retina during the point scan. The scanning system is therefore arranged to acquire A-scans at respective scan locations that are distributed across a surface of the retina, by sequentially illuminating the scan locations with the light beam, one scan location at a time, and collecting at least some of the light scattered by the retina at each scan location. The scanning system may acquire OCT images in the form of repeat B-scans by performing the point-scan using a linear scan pattern, wherein a set of overlapping scan lines are followed during the scan. Although the scanning system is arranged to acquire repeat B-scans by performing point-scans in the present example embodiment, the scanning system may alternatively be arranged to acquire the repeat B-scans by performing line-scans in other example embodiments, using hardware well-known to those versed in the art. The FD-OCT imaging system 30 may alternatively be arranged to acquire OCT images in the form of C-scans by performing point-scans or a line-scans using techniques well-known to those versed in the art, or by employing a full-field set-up.

The first OCT image mentioned above may, as in the present example embodiment, be in the form of a first OCT B-scan 10-1, and the second OCT image may be in the form of a second OCT B-scan 10-2, as shown in Figure 1. However, the form of the first and second OCT images is not so limited, and the first and second OCT images may be respective OCT C-scans, for example. The first B-scan 10-1 and the second B-scan 10-2 of the common portion of the retina may, as in the present example embodiment, be next to each other in a set of the B-scan in a sequence of OCT B-scans 10 or they may be separated by n intervening B-scans, where $n \geq 1$ but small enough for the B-scans to have a sufficiently high degree of phase correlation to one another, owing to the absence of significant relative movement of the eye 20 with respect to the FD-OCT imaging system 30.

As described in more detail below, the data processing apparatus 100 is arranged to process at least part of the phase component of the first B-scan 10-1 and at least part of the phase component of the second B-scan 10-2 to calculate a velocity profile P comprising values of velocity v whose variation with position in the velocity profile is indicative of a distribution of velocity v among points along the axial direction z in common portion of the retina, as illustrated in Figure 1, for example. The velocity profile P may, as in the present example embodiment, thus be a two-dimensional data structure in which values of the velocity v are associated with corresponding values of a position. The velocity profile P may more generally be a two-dimensional data structure in which values (of a calculated change in phase or optical path length, for example) that are indicative of the velocity v are associated with corresponding values of a position. The data processing apparatus 100 need not calculate velocity profiles P, however, and may be limited to calculating a respective indication of a tissue velocity v at a single common position along the axial direction z for each set of B-scans of the plurality of sets of two or more B-scans.

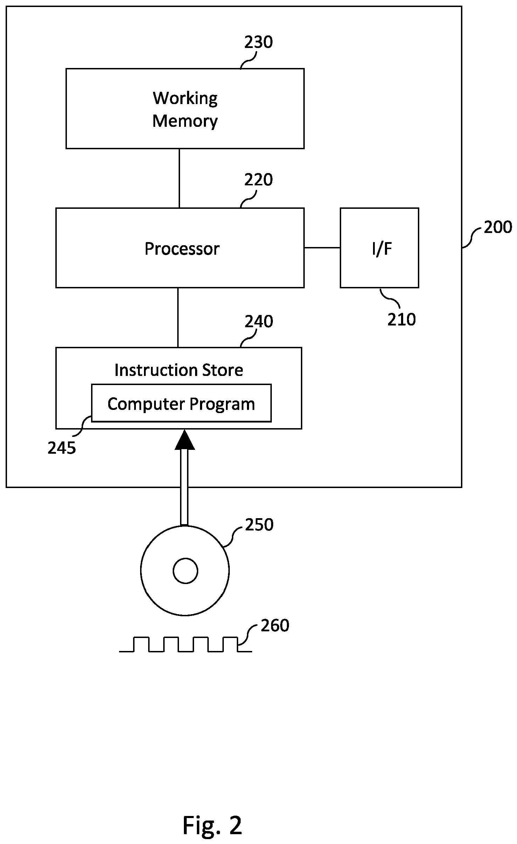

The data processing apparatus 100 may be provided in any suitable form, for example as a programmable signal processing hardware 200 of the kind illustrated schematically in Figure 2. The programmable signal processing hardware 200 comprises a communication interface (I/F) 210, for receiving the B-scans (or OCT images of another form, such as C-scans) from the FD-OCT imaging system 30, and outputting calculated indications of tissue velocity and/or a graphical representation thereof on a display, such a computer screen or the like. The signal processing hardware 200 further comprises a processor (e.g. a Central Processing Unit, CPU, and/or a Graphics Processing Unit, GPU) 220, a working memory 230 (e.g. a random-access memory) and an instruction store 240 storing a computer program 245 comprising the computer-readable instructions which, when executed by the processor 220, cause the processor 220 to perform the various functions of the data processing apparatus 100 described herein. The working memory 230 stores information used by the processor 220 during execution of the computer program 245. The instruction store 240 may comprise a ROM (e.g. in the form of an electrically erasable programmable read-only memory (EEPROM) or flash memory) which is pre-loaded with the computer-readable instructions. Alternatively, the instruction store 240 may comprise a RAM or similar type of memory, and the computer-readable instructions of the computer program 245 can be input thereto from a computer program product, such as a non-transitory, computer-readable storage medium 250 in the form of a CD-ROM, DVDROM, etc. or a computer-readable signal 260 carrying the computer-readable instructions. In any case, the computer program 245, when executed by the processor 220, causes the processor 220 to perform the functions of the data processing apparatus 100 described herein. Thus, the data processing apparatus 100 of the example embodiment may comprise a computer processor 220 and a memory 240 storing computer-readable instructions which, when executed by the processor 220, cause the processor 220 to process each set of OCT images of a plurality of different sets of OCT images in a sequence of OCT images 10 of a common portion of a retina of an eye 20 to generate a respective indication of a tissue velocity v at a position along an axial direction z in the OCT images 10 (i.e. the direction in any of the OCT images (e.g. B-scans or C-scans) along which the elements of a component A-scan of the OCT image are arrayed).

It should be noted, however, that the data processing apparatus 100 may alternatively be implemented in non-programmable hardware, such as an ASIC, an FPGA or other integrated circuit dedicated to performing the functions of the data processing apparatus 100 described herein, or a combination of such non-programmable hardware and programmable signal processing hardware 200 as described above with reference to Figure 2.

The data processing apparatus 100 may be provided as a stand-alone product or as part of a system 1000 comprising the FD-OCT imaging system 30, which is arranged to acquire the sequence of OCT images 10 of the common portion of the retina of the eye 20, typically both before and after stimulation of the common portion by an optical stimulus 40 generated by a light source 45 (e.g. an LED). The data processing apparatus 100 is arranged to process at least part of a phase component of a first OCT image 10-1 and at least part of a phase component of a second OCT image 10-2 of the sequence of OCT images 10 acquired by the FD-OCT imaging system 30 using the techniques described herein.

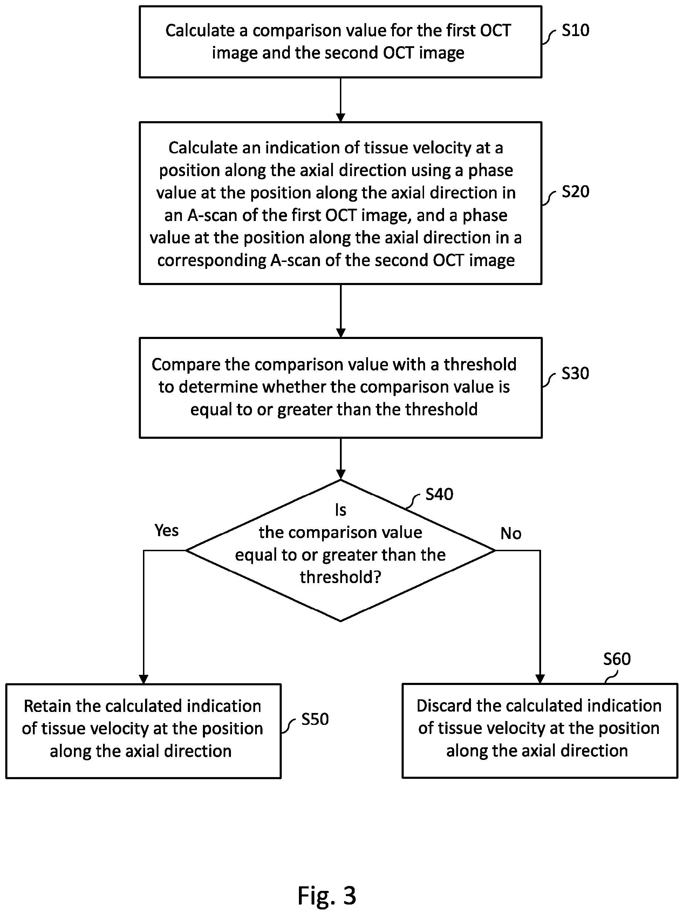

Figure 3 is a flow diagram illustrating a method by which the data processing apparatus 100 processes each set of B-scans, for example a pair of B-scans comprising the first B-scan 10-1 and the second B-scan 10-2, from a plurality of sets of B-scans in the sequence of B-scans 10 of the common portion of the retina shown in Figure 1, to generate a respective indication of a tissue velocity v at a position along an axial direction z in the B-scans 10.

In process S10 of Figure 3, the data processing apparatus 100 calculates a comparison value based on based on the first OCT image (B-scan 10-1 in the present example embodiment) and the second OCT image (B-scan 10-2 in the present example embodiment). The comparison value may be a value of an image quality metric or attribute calculated based on at least one of an amplitude component of the first B-scan 10-1 and an amplitude component of the second B-scan 10-2. The image quality metric (attribute) may take the form of a dynamic range, a contrast, a signal-to-noise ratio or a sharpness function value from a B-scan. A measure of the success of an intensity-based segmentation of a B-scan is another example of an image quality metric that may be used. Alternatively, the comparison value may be a value of an image similarity metric that provides a measure of a degree of similarity between images, the value of the image similarity metric being calculated based on the first B-scan 10-1 and the second B-scan 10-2. The image similarity metric may, as in the present example embodiment, take the form of a maximum value of the cross-correlation between the first B-scan 10-1 and the second B-scan 10-2. Other image similarity metrics that could alternatively be used include the sum of squared differences, mutual information, normalised mutual information, and Kullback Leibler distance, among others.

The cross-correlation between two complex functions $f(t)$ and $g(t)$ of a real variable t, denoted f*g, is defined by $f*g = \overline{f}(t) * g(t)$, where * denotes convolution, and $\overline{f}(t)$ is the complex conjugate of f(t).

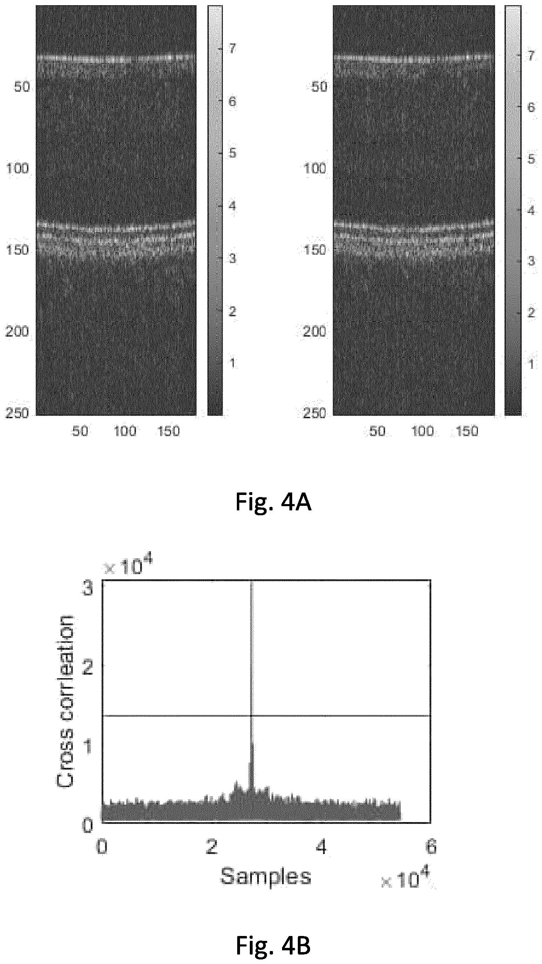

The maximum value of the cross-correlation between two B-scans is a good metric of how closely related the B-scans are to each other. For example, the two B-scans shown in Figure 4A are highly correlated to one another, and the calculated cross-correlation between these B-scans has a high peak value of about $3 \cdot 10^4$, as shown in Figure 4B.

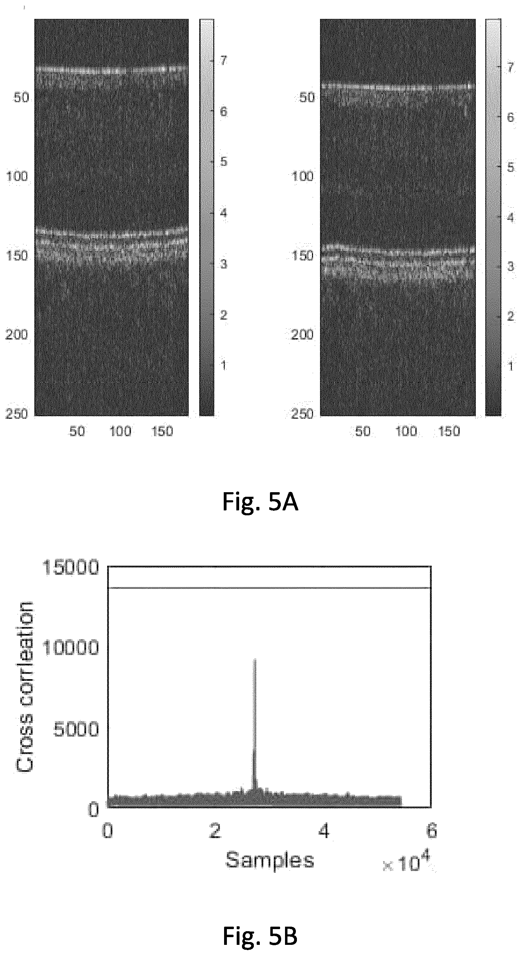

For comparison, Figure 5A shows two B-scans that are dissimilar. This dissimilarity may be caused by factors such as eye movement, for example, and would lead to bad image registration. For the example B-scans shown in Figure 5A, the maximum value of the calculated cross-correlation between the B-scans is much lower than for the B-scans shown in Figure 5A, and is about $1 \cdot 10^4$, as shown in Figure 5B.

Referring again to Figure 3, in process 520, the data processing apparatus 100 calculates an indication of tissue velocity v at a position along the axial direction z using a phase value at the position along the axial direction in an A-scan of the first OCT image (B-scan 10-1), and a phase value at the same position along the axial direction z in a corresponding A-scan of the second OCT image (B-scan 10-2). The corresponding A-scan of B-scan 10-2 has the same position along the x-axis of B-scan 10-2, along which its A-scans are arrayed, as the position of the A-scan considered in B-scan 10-1. By the processing of each set of the sets of B-scans in accordance with S20 of Figure 3, the data processing apparatus 100 may calculate a respective indication of tissue velocity v at a single common position along the axial direction z for each of the sets of B-scans or, as in the present example embodiment, a respective velocity profile P for each of the sets of B-scans, which is indicative of a distribution, along the axial direction z, of tissue velocity v within the common portion of the retina.

In more detail, in process S20 of Figure 3, the data processing apparatus 100 processes the phase component of the first B-scan 10-1 and the phase component of the second B-scan 10-2 to calculate a velocity profile P indicative of a distribution, along the axial direction z, of velocity v within the common portion of the retina. The data processing apparatus 100 thus calculates a velocity profile P comprising values of velocity v, or values of a change in phase or optical path length, $\Delta OPL$, for example, whose variation with position in the velocity profile P is indicative of a distribution of velocity v of the retina (within the common portion) at respective points lying on a line aligned with the axial direction. The velocity profile P is thus a two-dimensional data structure in which values of the tissue velocity v (or values of a variable indicative thereof, such as $\Delta OPL$ or a change in phase, $\Delta\phi$) are associated with corresponding values of the position, and this data structure may be visualised in the form of a plot of velocity v (or $\Delta\phi$ or $\Delta OPL$) as a function of position z, as shown schematically in Figure 1.

Before calculating the indication of tissue velocity v in process S20 of Figure 3, the data processing apparatus 100 may process the phase value at the position along the axial direction z in the A-scan of the first B-scan 10-1, and the phase value at the position along the axial direction z in the corresponding A-scan of the second B-scan 10-2, to compensate for a bulk motion of the common portion of the retina during acquisition of the sequence of OCT images 10 by the Fourier-domain OCT imaging system 30.

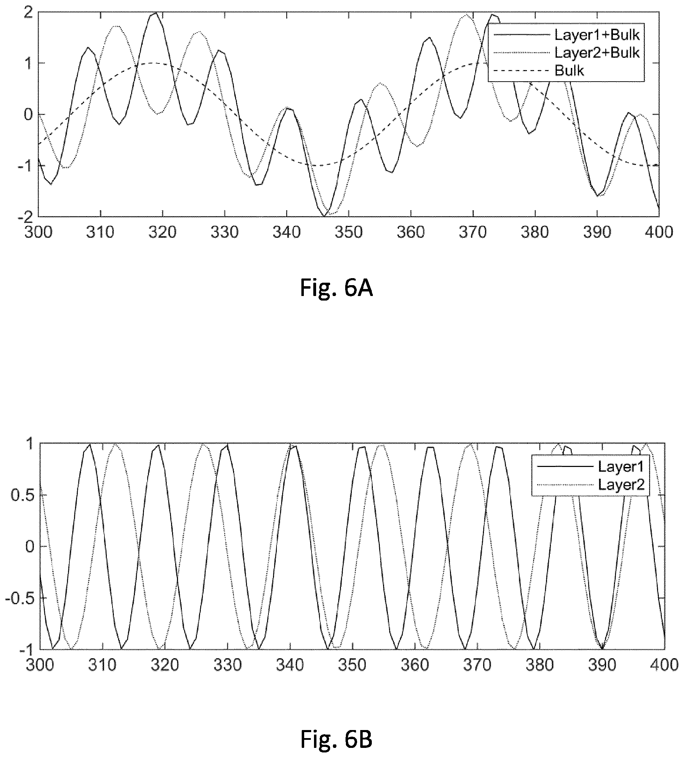

Figure 6A illustrates a sinusoidal bulk oscillation added to oscillatory movements of two model retinal layers. In this example, the bulk movement is modelled by adding a phase $\exp(i\varphi)$, where $\varphi$ represents a fraction of a wavelength (half wavelength movement is $2\pi$ in double pass reflection). Figure 6A shows how the phase angle of light reflected from the first model retinal layer (Layer 1) and the second model retinal layer (Layer 2) varies over time whilst the layers are subject to the common bulk motion as well as their individual oscillations (i.e. the plots labelled "Layer 1 + Bulk" and "Layer 2 + Bulk" in Figure 6A). Figure 6A also shows how the phase angle of light reflected from either model retinal layer varies over time whilst the layer is subject to the bulk motion only (i.e. the plot labelled "Bulk" in Figure 6A). Figure 6B shows the effect of removing the bulk motion on the variation of the phase angle of light reflected from the two model retinal layers with time.

The MATLAB™ code used to generate the plots in Figures 6A and 6B is as follows:

```
t=(0:1000);
A=0.95;
B=0.8;

RetinaBulkMovement=exp(j*cos(t*pi/sqrt(700)));
Layer1Movement=exp(j*cos(t*pi/sqrt(30)));
Layer2Movement=exp(j*cos(t*pi/sqrt(50)));

Layer1totalMovement=Layer1Movement.*RetinaBulkMovement;
Layer2totalMovement=Layer2Movement.*RetinaBulkMovement;

%%%%%%%%%%%%%%%%

Layer1MovementDeduced=angle(Layer1totalMovement.*conj(RetinaBulkMovement));

Layer2MovementDeduced=angle(Layer2totalMovement.*conj(RetinaBulkMovement));

subplot(211);
plot(angle(Layer1totalMovement));hold
on;plot(angle(Layer2totalMovement));plot(angle(RetinaBulkMovement));legend('Layer1+
Bulk','Layer2+Bulk','Bulk');
xlim([300 400]);

subplot(212);
plot(Layer1MovementDeduced);hold on;plot(Layer2MovementDeduced);shg;hold off;
xlim([300 400]);
legend('Layer1','Layer2');
```

An example of a process by which the data processing apparatus 100 may perform S20 of Figure 3 will now be described, which is based on the velocity-based ORG technique described in Kari V. Vienola, et al., "Velocity-based optoretinography for clinical applications," Optica 9, 1100-1108 (2022), the content of which is herein incorporated by reference in its entirety.

The data processing apparatus 100 may, as in the present example embodiment, first flatten the first B-scan 10-1 and the second B-scan 10-2 such that the IS/OS and COST reflections lie at substantially the same height for each A-scan in each of the B-scans. The data processing apparatus 100 may then register the second B-scan 10-2 with respect to the first B-scan 10-1. The phase data of the two B-scans for each spatial coordinate pair (i.e. the phase data at the same (x, z) coordinates in the B-scans) may then be unwrapped in the temporal dimension to minimise the magnitude of the difference in phase between the data sets of the two B-scans 10-1 and 10-2. After unwrapping and processing the phase components of the first B-scan 10-1 and the second B-scan 10-2 to compensate for the bulk motion of the retina during imaging, a difference between corresponding phase values in the B-scans 10-1 and 10-2 is calculated for each spatial location specified by a corresponding coordinate pair, and the instantaneous velocity for the spatial location is then calculated using the difference between the acquisition times of the B-scans. These instantaneous velocities may, as in the present example embodiment, be averaged in the lateral dimension (i.e. along the x-axis) to give instantaneous, depth-dependent measures of velocity along the z-axis, i.e. a one-dimensional velocity profile P of the kind illustrated schematically in Figure 1, which is indicative of a distribution of velocity v along the axial direction (z-axis) within the common portion of the retina that is covered by the first and second B-scans 10-1 and 10-2. The B-scan amplitudes (if desired) can also be averaged in the lateral dimension (x-axis) to give instantaneous, depth-dependent measures of backscattering. It should be noted that averaging in the lateral dimension is not required, and a two-dimensional velocity profile may alternatively be generated, which is indicative of the distribution of velocity v along both the axial direction (z-axis) and the lateral direction (x-axis) within the common portion of the retina that is covered by the first B-scan 10-1 and the second B-scan 10-2.

The tissue velocity v in a portion of the velocity profile P corresponding to the photoreceptor OS in the retina varies (usually monotonically) from a maximum velocity $v_{max}$ at a first position $z_{max}$ in the velocity profile P corresponding to the IS-OS junction at a given location on the retina to a minimum velocity $v_{min}$ at a second position $z_{min}$ in the velocity profile P corresponding to the COST at that location on the retina, as illustrated schematically in Figure 1.

The velocity profiles P (or the indications of tissue velocity v at a single common position along the axial direction z in other example embodiments) calculated by the processing of OCT images in each set of OCT images from the sequence of OCT images 10 in accordance with process S20 of Figure 2 are useful for a variety of purposes. For example, the velocity profiles (or the aforementioned indications of tissue velocity) may be concatenated and a cumulative sum of values in the resulting concatenation may be calculated to generate ORG data indicative of the response to the retina to an applied optical stimulus, as described below. Additionally or alternatively, the velocity profiles (or the aforementioned indications of tissue velocity) may be processed to determine the position(s) of one or both of the boundaries of the OS or other layer L of the retina, which position(s) change in response to applied optical stimuli (for example, the position of the rod outer segment tips (ROST) or the retina pigment epithelium (RPE)), using the velocity-based approach to segmentation described in co-pending European application bearing reference number 253 989. For example, the velocity profile P may have a further maximum followed by a further minimum going along the z-axis in Figure 1 for z > $z_{min}$, which corresponds to a change in length of rods in response to the applied stimulus. The data processing apparatus 100 may, as in the present example embodiment, determine both the first position $z_{max}$ and the second position $z_{min}$ in at least one of the calculated velocity profiles P by firstly calculating, for each combination of a candidate first position, $z_i$, in the velocity profile P and a candidate second position, $z_j$, in the velocity profile P of all combinations ($z_i$, $z_j$) of candidate first and second positions, a respective value of a difference, $D_{ij}$, between a respective velocity $v_i$ at the candidate first position $z_i$ and a respective velocity $v_j$ at the candidate second position $z_j$ in the combination ($z_i$, $z_j$). The data processing apparatus 100 then identifies, as the first position $z_{max}$ and the second position $z_{min}$, a candidate first position $z_i$ and a candidate second position $z_j$ of a combination in the plurality of combinations for which the calculated value of the difference $D_{ij}$ is greatest among the calculated values of the difference.

Once the boundaries of the OS have been identified as described above, their respective velocities can be extracted from the velocity profile P, and the difference between the extracted velocities provides the rate of the contraction/elongation of the OS at the time the B-scans 10-1 and 10-2 were acquired by the FD-OCT imaging system 30, which can be used to generate ORG data indicative of the response of the retina in the common portion to the applied stimulus.

For whichever purpose the velocity profiles P or the aforementioned indications of tissue velocity are subsequently used, it is useful to gauge how reliable they are and to remove any unreliable velocity profiles (or unreliable indications of tissue velocity) from the processing pipeline, to prevent these from adversely affecting the ORG data or other final result. The reliability of the velocity profiles P or the aforementioned indications of tissue velocity will be influenced by the image quality of the first B-scan 10-1 and the second B-scan 10-2, and how closely correlated the B-scans are to each other. The maximum value of a calculated cross-correlation between the two B-scans provides a good metric of how closely correlated the B-scans are, and therefore the reliability of the velocity profiles P or of the aforementioned indications of tissue velocity determined by the data processing apparatus 100.

Referring again to Figure 3, in process 530, the data processing apparatus compares the comparison value calculated in process S10 of Figure 3 with a threshold to determine whether the comparison value is equal to or greater than the threshold. In the present example embodiment, some pairs of B-scans yield comparison values that are greater than or equal to the threshold, and some other pairs of B-scans yield comparison values that are smaller than the threshold. Incidentally, it is noted that although process S20 of Figure 3 is illustrated and described herein as being performed after process 510, the order in which these processes are performed may be reversed or they may be performed concurrently. Alternatively, process S20 may be performed concurrently with process S30 in Figure 3.

The value of the threshold in process S30 may depend on the use that is subsequently made of the velocity profiles P or the aforementioned indications of tissue velocity, and may be chosen by trial and error such that retention of only the velocity profiles P (or the aforementioned indications of tissue velocity) that are associated with comparison values that equal or exceed the chosen threshold reduce or prevent a degradation of the ORG data or other processing results that would otherwise occur.

For example, where one or more of the velocity profiles are used in the velocity-based segmentation technique described in co-pending European application bearing reference number 253 989, the threshold may be set by comparing the segmentation results from the data processing apparatus 100 (i.e. the boundaries of the layer L determined by the data processing apparatus 100) with a layer segmentation that results from the user's inspection of the source B-scans, whilst having regard to maximum cross-correlation values calculated for the source B-scans. In this way, it may be determined that pairs of B-scans, for which the maximum value of a cross-correlation calculated therebetween is below a certain threshold, tend to yield unreliable values for the indications of the layer boundaries when the B-scans are processed by the data processing apparatus 100 as described herein, whereas pairs of B-scans, for which the maximum value of a cross-correlation calculated therebetween is at or above that threshold, tend to yield reliable values for the indications of the layer boundaries.

For example, it may be found that the B-scans shown in Figure 4A yield indications of the layer boundaries that compare favourably with the results of a manual segmentation of the OS performed by inspection of these B-scans, whereas the B-scans shown in Figure 5A yield indications of the layer boundaries that differ significantly from the results of a manual segmentation of the OS performed by inspection of these B-scans. In this case, it may be appropriate to set the threshold to a value between the maximum values of the cross-correlation shown in Figures 4B and 5B (i.e. about $3 \cdot 10^4$ and $1 \cdot 10^4$, respectively), for example to a value of about $1.4 \cdot 10^4$, as shown by the horizontal black line in Figure 5B. B-scans that yield a maximum cross-correlation value below this threshold may be regarded as incapable of allowing reliable segmentation to be performed.

In case the comparison value is determined in process S30 of Figure 3 to be equal to or greater than the threshold ("Yes" at S40 in Figure 3), the data processing apparatus 100 retains (stores) the velocity profile P or, more generally, the indication of tissue velocity v at the position along the axial direction z that is calculated in process S20 of Figure 3.

On the other hand, in case the comparison value is determined in process S30 of Figure 3 not to be equal to or greater than the threshold, the data processing apparatus 100 discards the velocity profile P or, more generally, the indication of tissue velocity v at the position along the axial direction z that is calculated in process S20 of Figure 3, and therefore does not use it in subsequent processing operations. The data processing apparatus 100 may erase or overwrite the calculated indication (or velocity profile, as the case may be), for example. Where a velocity profile P is calculated in process S20 of Figure 3, as in the present example embodiment, the data processing apparatus 100 may, following the negative determination in S40 of Figure 3, retain in storage (i.e. memory) for subsequent use, in place of the calculated velocity profile P (or, more generally, the calculated indication of tissue velocity at the position along the axial direction z), a null velocity profile which indicates zero velocity v at all positions along the axial direction z or, in the more general case, an indication of zero tissue velocity at the position along the axial direction.

Following process S50 of Figure 3, the data processing apparatus 100 may, as in the present example embodiment, concatenate the retained velocity profiles P or, more generally, the retained indications of tissue velocity that have been calculated in process S20 of Figure 3, together with any null velocity profiles (or indications of zero tissue velocity in the more general case) generated following a negative determination in S40 of Figure 3. The resulting concatenation is indicative of how the tissue velocity v changes over time.

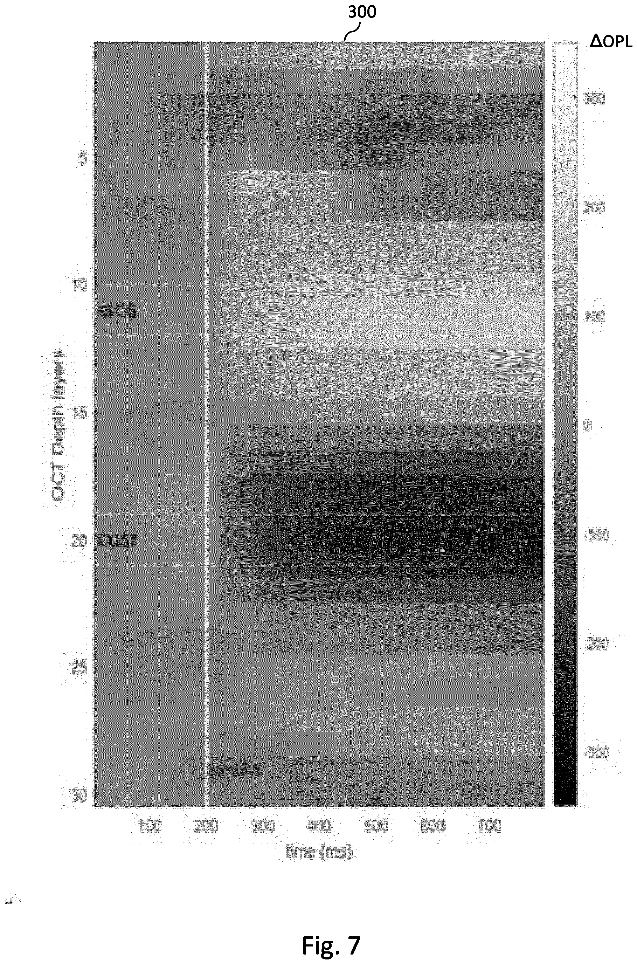

Figure 7 shows an example of a concatenation 300 of the velocity profiles P generated by the data processing apparatus 100. Each velocity profile P extends along the y-axis (labelled "OCT depth layers") in Figure 7, and the velocity profiles calculated for adjacent pairs of B-scans in the sequence of B-scans 10 are arrayed along the x-axis (labelled "time (ms)") in Figure 7. Figure 7 shows the changes in optical path length, ΔOPL, (which provides an indication of the velocity v) with position in each velocity profile P, which is derived from B-scans that have been acquired after application of the optical stimulus at time t = 200 ms. The change in optical path length ΔOPL varies with position in each velocity profile (i.e. along the y-axis in Figure 7) from a minimum ΔOPL of about -300 (arb. unit) to a maximum ΔOPL of about +250 (arb. unit).

The data processing apparatus 100 may, in the present example embodiment, also integrate respective portions of velocity profiles P in the concatenation of the velocity profiles, which portions have the same position along the axial direction z to generate ORG data indicating an optical path length variation over time at the position along the axial direction z. The data processing apparatus 100 may, more generally, integrate (i.e. calculate a cumulative sum or running total of) the indications of tissue velocity at the position that have been retained in process S50 of Figure 3, and any substitute indications of zero velocity that may have been generated following a negative determination in S40 of Figure 3, to generate ORG data indicating an optical path length variation over time at the position along the axial direction z. The data processing apparatus 100 may process each velocity profile P in the concatenated set of velocity profiles by selecting a portion (segment or data element) of the velocity profile P that is disposed at a predetermined position along the axial direction z of the velocity profile P, and then integrate the selected (commonly located) portions by calculating a cumulative sum (or running total) of the velocity values in these portions, which indicates how an optical path length of an optical path of an OCT sample beam that ends at a location in the retina corresponding to the predetermined position in the velocity profile P changes over time.

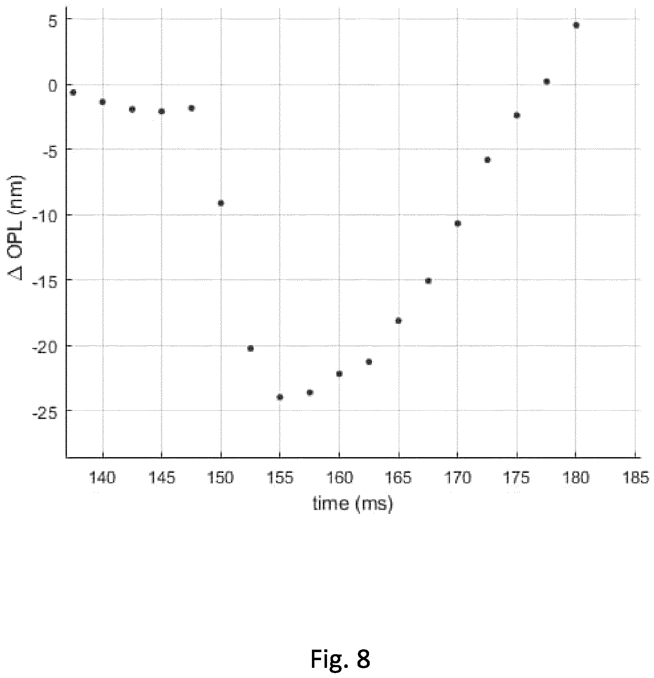

Figure 8 shows a plot of changes in optical path length $\Delta$OPL over time, which have been calculated by the data processing apparatus 100 for a selected position in the velocity profiles of the concatenation of velocity profiles. The plot in Figure 8 shows how $\Delta$OPL at the common position changes from one B-scan to the next in the sequence of B-scans 10.

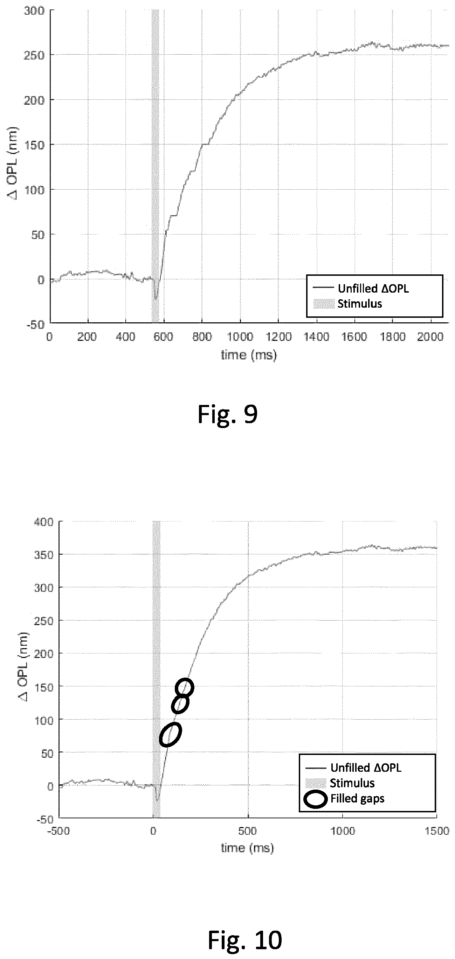

Figure 9 shows a plot of cumulative changes in the optical path length over time, which is known as the ORG signal (or ORG data). The ORG signal is obtained by calculating a cumulative sum of optical path length changes for a selected position in the velocity profiles of the concatenation of velocity profiles. As shown in Figure 9, the ORG signal becomes negative shortly after the stimulus is applied, and then increases to become positive, eventually levelling off at a value of approximately 250 nm in this example. Figure 9 also shows the ORG signal to have several plateaus, which are caused by the replacements of velocity profiles calculated using B-scans that do not yield a sufficiently high maximum cross-correlation value with velocity profiles that indicate zero velocity (i.e. null velocity profiles), as described above.

The ORG data may thus comprise one or more sets of equal consecutive values, and the data processing apparatus 100 may be arranged to smooth the generated ORG data by replacing one or more values in at least one set of the one or more sets of equal consecutive values with one or more estimated values calculated based on neighbouring values that neighbour the set of equal consecutive values in the ORG data. The data processing apparatus 100 may perform this smoothing by using a moving median or a moving mean, for example, where a median or mean of a specified number of points either side of the missing data point(s) is calculated and then assigned to the missing point(s). Alternatively, the data processing apparatus 100 may, for example, detect each set of equal consecutive values in the ORG data, and replace the values in each detected set with corresponding estimated values that are obtained by interpolating (e.g. linearly) between a first value in the ORG data which is adjacent to the first of the equal consecutive values in the detected set, and a second value in the ORG data which is adjacent to the last of the equal consecutive values in the detected set.

A result of smoothing the ORG signal using a moving mean is shown in Figure 10. Before the smoothing has been applied, large portions of the data represent OPL changes of zero, as shown in Figure 9, and thus generally underrepresent actual values that would be observed. Using the method described herein, the equal values are replaced with estimated values (as indicated by circles in Figure 10), which represent the change in OPL more accurately.

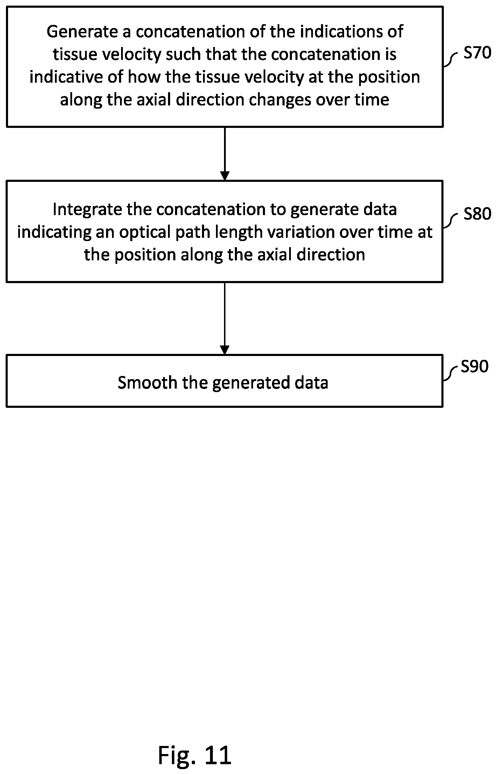

The optional further processing that may be performed by the data processing apparatus 100 of the present example embodiment is summarised in Figure 11.

In process S70 of Figure 11, the data processing apparatus 100 generates a concatenation 300 of the generated indications of tissue velocity v such that the concatenation 300 is indicative of how the tissue velocity v at the position along the axial direction z changes over time.

In process S80 of Figure 11, the data processing apparatus 100 integrates the concatenation 300 to generate data indicating an optical path length variation over time at the position along the axial direction z.

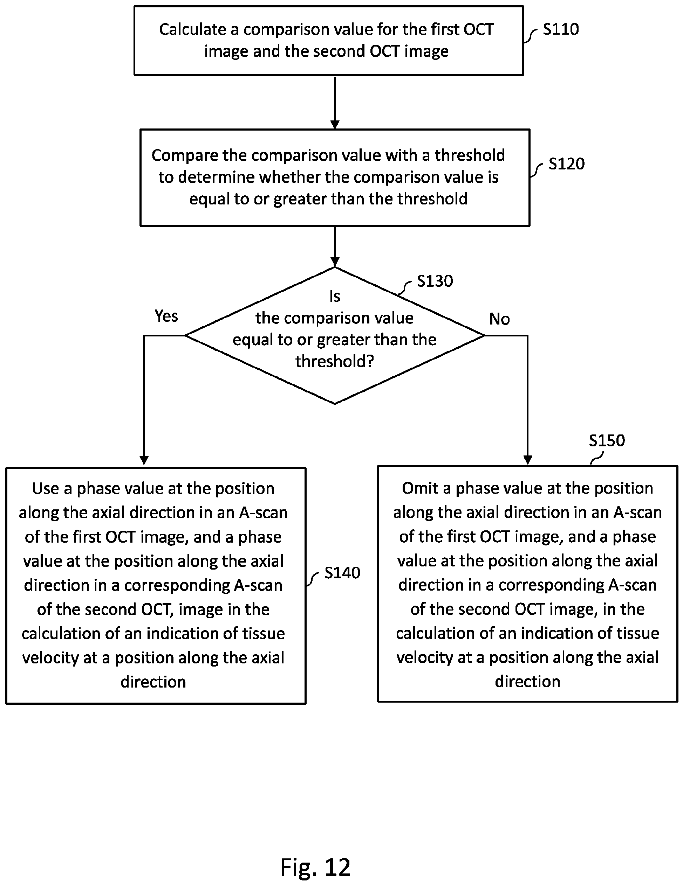

Where the comparison value is determined in process S40 of Figure 3 (or process 5130 in Figure 12 of the second example embodiment described below) not to be equal to or greater than the threshold, the respective indication of tissue velocity v at the position along the axial direction z may, as in the present example embodiment, be set to indicate zero velocity, and the generated data may comprise one or more sets of equal consecutive values, as described above.

In process S90 of Figure 11, the data processing apparatus 100 smooths the generated data by replacing one or more values in a set of the one or more sets of equal consecutive values with one or more estimated values calculated based on neighbouring values that neighbour the set of equal consecutive values in the generated data.

Although the data processing apparatus 100 processes pairs of adjacent B-scans in the sequence of B-scans 10 in turn (i.e. one pair after another adjacent pair in the sequence) to generate the velocity profiles P or, more generally, the indications of tissue velocity at the position along the axial direction z, the data processing apparatus 100 may alternatively generate these by processing pairs of adjacent B-scans in parallel, thereby greatly speeding up the process. Furthermore, although pairs of B-scans that are adjacent to each other in the sequence of B-scans 10 (i.e. consecutive B-scans in the sequence) are processed, the data processing apparatus 100 may alternatively process pairs of B-scans in the sequence where the B-scans of each pair are separated from each other by one or more intervening B-scans, for example.

Further still, although the data processing apparatus 100 of the present example embodiment has been described as processing pairs of B-scans, it may more generally be configured to process sets of more than two B-scans in the sequence of B-scans 10, which B-scans may be consecutive B-scans in the sequence or individual B-scans in the sequence that are separated from each other by one or more intervening B-scans not forming part of the set. The respective sets of (e.g. 5) B-scans may be selected by sliding a window selection function along the sequence of B-scans 10, where, in each performance of the process of Figure 3, the window selection function selects all B-scans in a windowed portion of the sequence of B-scans 10 or every other B-scan in the windowed portion, for example, and the selected sets may have one or more B-scans in common.

In the modified form of process S10 in Figure 3, the data processing apparatus 100 calculates a comparison value for a set of a plurality of different sets of consecutive B-scans in the sequence of B-scans 10. The comparison value may take any of the different forms described above. The data processing apparatus 100 then processes phase components of at least some of the B-scans in the set in accordance with a modified form of process S20 of Figure 3, to generate a velocity profile P that is indicative of the distribution or, more generally, an indication of tissue velocity v at a position along the axial direction z. Here, the data processing apparatus 100 may first flatten the B-scans of the set such that the IS/OS and COST reflections lie at substantially the same height for each A-scan in each of the B-scans. The data processing apparatus 100 may then register the B-scans with respect to each other. The phase data cube $\theta(x, z, t)$ is then unwrapped in the temporal dimension by adding or subtracting $2\pi$ to $\theta(x_p, z_q, t_r)$ to minimise $|\theta(x_p, z_q, t_r) - \theta(x_p, z_q, t_{r-1})|$, where $t_r$ and $t_{r-1}$ represent consecutive phase B-scans. This step is performed for each spatial coordinate pair $(x_p, z_q)$ in the volume. A rate of phase change is computed for each coordinate pair by performing a least-squares linear fit with respect to t, giving $\frac{\Delta\theta}{\Delta t}(x, z)$ in

rad/s. From this, the instantaneous velocity for each spatial location may be calculated as
$$\frac{\Delta z}{\Delta t}(x, z) = \frac{\Delta\theta}{\Delta t}(x, z) \cdot \frac{\lambda}{4\pi n},$$
where $\lambda$ is the wavelength of OCT light being used, and $n$ is a nominal refractive index of the eye 20. These instantaneous velocities may be averaged in the lateral dimension (i.e. along the x-axis) to give instantaneous, depth-dependent measures of velocity along the z-axis, i.e. a one-dimensional velocity profile P of the kind illustrated schematically in Figure 1, which is indicative of a distribution of velocity v along the axial direction (z-axis) within the common portion of the retina that is covered by the repeat B-scans. The B-scan amplitudes (if desired) can also be averaged in the lateral dimension (x-axis) to give instantaneous, depth-dependent measures of backscattering. It should be noted that averaging in the lateral dimension is not required, and a two-dimensional velocity profile may alternatively be generated, which is indicative of the distribution of velocity v along both the axial direction (z-axis) and the lateral direction (x-axis) within the common portion of the retina that is covered by the repeat B-scans. The process then proceeds in accordance with S30 o S60 of Figure 3.

[Second Example Embodiment]

In the first example embodiment described above, a respective indication of tissue velocity at one or more positions along the axial direction z is calculated for each set of a plurality of sets of B-scans in the sequence of B-scans 10, and each calculated indication is either retained or discarded, depending on how the associated comparison value compares with the threshold. However, in the present example embodiment, calculations of the indications of tissue velocity v are performed selectively (i.e. not necessarily for every set of B-scans), depending on how comparison values calculated for the sets of OCT images compare with the threshold, as will now be described in more detail with reference to Figure 12.

Figure 12 is a flow diagram illustrating a method by which the data processing apparatus 100 of the present example embodiment processes each set of B-scans, for example a pair of B-scans comprising the first B-scan 10-1 and the second B-scan 10-2, from a plurality of sets of B-scans in the sequence of B-scans 10 of the common portion of the retina shown in Figure 1, to generate a respective indication of a tissue velocity v at a position along an axial direction z in the B-scans 10.

Processes S110 to S130 of Figure 12 are the same as processes S10, S30 and S40 of Figure 3, respectively, which have been described in detail above.

Where the data processing apparatus 100 determines the comparison value to be equal to or greater than the threshold ("Yes" at S130 in Figure 12), in process S140 of Figure 12, the data processing apparatus 100 uses a phase value of an OCT data element at the position along the axial direction z in an A-scan of the first B-scan 10-1 in the set, and a phase value at the same position along the axial direction z in a corresponding A-scan of the second B-scan 10-2 in the set, in a calculation of the respective indication of tissue velocity v at that position along the axial direction z.

Where the data processing apparatus 100 determines the comparison value not to be equal to or greater than the threshold ("No" at S130 in Figure 12), in process S150 of Figure 12, the data processing apparatus 100 omits the phase value at the position along the axial direction z in the A-scan of the first B-scan 10-1 in the set, and the phase value at the same position along the axial direction z in the corresponding A-scan of the second B-scan 10-2 in the set, in the calculation of the respective indication of tissue velocity v at that position along the axial direction z. The data processing apparatus 100 may, following the negative determination in S130 of Figure 12, generate a null velocity profile which indicates zero velocity v at all positions along the axial direction z or, in the more general case, an indication of zero tissue velocity at the position along the axial direction.

Similar to the first example embodiment, the data processing apparatus 100 may then concatenate the calculated velocity profiles P or, more generally, the calculated indications of tissue velocity that have been calculated in process S140 of Figure 12, together with any null velocity profiles (or indications of zero tissue velocity in the more general case) generated following a negative determination in S130 of Figure 12. The resulting concatenation is indicative of how the tissue velocity v changes over time.

The data processing apparatus 100 may, in the present example embodiment, also integrate respective portions of velocity profiles P in the concatenation of the velocity profiles, which portions have the same position along the axial direction z (or, more generally, integrate the indications of tissue velocity that have been calculated in process S140 of Figure 12 and any indications of zero velocity that may have been generated following a negative determination in S130 of Figure 12), to generate ORG data indicating an optical path length variation over time at the position along the axial direction z. The data processing apparatus 100 may process each velocity profile P in the concatenated set of velocity profiles by selecting a portion (segment or data element) of the velocity profile P that is disposed at a predetermined position along the axial direction z of the velocity profile P, and then integrate the selected (commonly located) portions by calculating a cumulative sum (or running total) of the velocity values in these portions, which indicates how an optical path length of an optical path of an OCT sample beam that ends at a location in the retina corresponding to the predetermined position in the velocity profile P changes over time.

The ORG data may thus comprise one or more sets of equal consecutive values, and the data processing apparatus 100 may be arranged to smooth the generated ORG data by replacing one or more values in at least one set of the one or more sets of equal consecutive values with one or more estimated values calculated based on neighbouring values that neighbour the set of equal consecutive values in the ORG data. The data processing apparatus 100 may perform this smoothing by using a moving median or a moving mean, for example, where a median or mean of a specified number of points either side of the missing data point(s) is calculated and then assigned to the missing point(s). Alternatively, the data processing apparatus 100 may, for example, detect each set of equal consecutive values in the ORG data, and replace the values in each detected set with corresponding estimated values that are obtained by interpolating (e.g. linearly) between a first value in the ORG data which is adjacent to the first of the equal consecutive values in the detected set, and a second value in the ORG data which is adjacent to the last of the equal consecutive values in the detected set.

It will be appreciated that at least some of the modifications of the first example embodiment described above may also be made to the present example embodiment.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein, such as the processing methods described with reference to Figures 3, 11 and 12, may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, floppy diskettes, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein. Furthermore, it is common in the art to speak of software, in one form or another (e.g., program, procedure, process, application, module, unit, logic, and so on), as taking an action or causing a result. Such expressions are merely a shorthand way of stating that the execution of the software by a processing system causes the processor to perform an action to produce a result.

Some or all of the functionality of the OCT data processing hardware 130 may also be implemented by the preparation of application-specific integrated circuits, field-programmable gate arrays, or by interconnecting an appropriate network of conventional component circuits.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While various example embodiments of the present invention have been described above, it should be understood that they have been presented by way of example, and not limitation. It will be apparent to persons skilled in the relevant art(s) that various changes in form and detail can be made therein. Thus, the present invention should not be limited by any of the above-described example embodiments, but should be defined only in accordance with the following claims and their equivalents.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

Having now described some illustrative embodiments and embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

**Claims**

1.  A computer-implemented method of processing each set of optical coherence tomography, OCT, images comprising a first OCT image (10-1) and a second OCT image (10-2) of a plurality of different sets of OCT images in a sequence of OCT images (10) of a common portion of a retina of an eye (20) acquired by a Fourier-domain OCT imaging system (30) to generate a respective indication of a tissue velocity (v) at a position along an axial direction (z) in the OCT images (10), the method comprising performing, for each set of the sets of OCT images:

    calculating (S10) a respective comparison value being one of:

    a value of an image quality metric calculated based on at least one of the first OCT image (10-1) in the set and the second OCT image (10-2) in the set; and
    a value of an image similarity metric that provides a measure of a degree of similarity between images, the value of the image similarity metric being calculated based on the first OCT image (10-1) in the set and the second OCT image (10-2) in the set;

    calculating (S20) the respective indication of tissue velocity (v) at the position along the axial direction (z) using a phase value at the position along the axial direction (z) in an A-scan of the first OCT image (10-1) in the set, and a phase value at the position along the axial direction (z) in a corresponding A-scan of the second OCT image (10-2) in the set;
    comparing (S30) the comparison value with a threshold to determine whether the comparison value is equal to or greater than the threshold;
    in case the comparison value is determined to be equal to or greater than the threshold, retaining (S50) the respective indication of tissue velocity (v) at the position along the axial direction (z); and
    in case the comparison value is determined not to be equal to or greater than the threshold, discarding (S60) the respective indication of tissue velocity (v) at the position along the axial direction (z).

2.  A computer-implemented method of processing each set of optical coherence tomography, OCT, images comprising a first OCT image (10-1) and a second OCT image (10-2) of a plurality of different sets of OCT images in a sequence of OCT images (10) of a common portion of a retina of an eye (20) acquired by a Fourier-domain OCT imaging system (30) to generate a respective indication of a tissue velocity (v) at a position along an axial direction (z) in the OCT images (10), the method comprising performing, for each set of the sets of OCT images:

calculating (S110) a respective comparison value being one of:

a value of an image quality metric calculated based on at least one of the first OCT image (10-1) in the set and the second OCT image (10-2) in the set; and
a value of an image similarity metric that provides a measure of a degree of similarity between images, the value of the image similarity metric being calculated based on the first OCT image (10-1) in the set and the second OCT image (10-2) in the set;

comparing (S120) the comparison value with a threshold to determine whether the comparison value is equal to or greater than the threshold;
in a case the comparison value is determined to be equal to or greater than the threshold, using (S140) a phase value at the position along the axial direction (z) in an A-scan of the first OCT image (10-1) in the set, and a phase value at the position along the axial direction (z) in a corresponding A-scan of the second OCT image (10-2) in the set, in a calculation of the respective indication of tissue velocity (v) at the position along the axial direction (z); and
in case the comparison value is determined not to be equal to or greater than the threshold, omitting (S150) a phase value at the position along the axial direction (z) in an A-scan of the first OCT image (10-1) in the set, and a phase value at the position along the axial direction (z) in a corresponding A-scan of the second OCT image (10-2) in the set, in the calculation of the respective indication of tissue velocity (v) at the position along the axial direction (z).

3. The computer-implemented method according to Claim 1 or Claim 2, wherein the comparison value is a maximum value of a calculated cross-correlation between the first OCT image (10-1) and the second OCT image (10-2).

4. The computer-implemented method according to any of Claims 1 to 3, further comprising:

generating (S70) a concatenation (300) of the generated indications of tissue velocity (v) such that the concatenation (300) is indicative of how the tissue velocity (v) at the position along the axial direction (z) changes over time; and
integrating (S80) the concatenation (300) to generate data indicating an optical path length variation over time at the position along the axial direction (z).

5. The computer-implemented method according to Claim 4, wherein

in the case where the comparison value is determined not to be equal to or greater than the threshold, the respective indication of tissue velocity (v) at the position along the axial direction (z) is set to indicate zero velocity, the generated data comprises one or more sets of equal consecutive values, and
the method further comprises smoothing (S90) the generated data by replacing one or more values in a set of the one or more sets of equal consecutive values with one or more estimated values calculated based on neighbouring values that neighbour the set of equal consecutive values in the generated data.

6. The computer-implemented method according to any preceding claim, further comprising processing the phase value at the position along the axial direction (z) in the A-scan of the first OCT image (10-1) in the set, and the phase value at the position along the axial direction (z) in the corresponding A-scan of the second OCT image (10-2) in the set, before the calculation of the respective indication of tissue velocity (v) at the position along the axial direction (z), to compensate for a bulk motion of the common portion of the retina during acquisition of the sequence of OCT images (10) by the Fourier-domain OCT imaging system (30).

7. A computer program (245) comprising computer-readable instructions which, when executed by a processor (220), cause the processor (220) to perform a method according to any of Claims 1 to 6.

8. A data processing apparatus (100) arranged to process each set of optical coherence tomography, OCT, images comprising a first OCT image (10-1) and a second OCT image (10-2) of a plurality of different sets of OCT images in a sequence of OCT images (10) of a common portion of a retina of an eye (20) acquired by a Fourier-domain OCT imaging system (30) to generate a respective indication of a tissue velocity (v) at a position along an axial direction (z) in the OCT images (10), the data processing apparatus (100) being arranged to perform, for each set of the sets of OCT images, processes of:

calculating a respective comparison value being one of:

a value of an image quality metric calculated based on at least one of the first OCT image (10-1) in the set and the second OCT image (10-2) in the set; and

a value of an image similarity metric that provides a measure of a degree of similarity between images, the value of the image similarity metric being calculated based on the first OCT image (10-1) in the set and the second OCT image (10-2) in the set;

calculating the respective indication of tissue velocity (v) at the position along the axial direction (z) using a phase value at the position along the axial direction (z) in an A-scan of the first OCT image (10-1) in the set, and a phase value at the position along the axial direction (z) in a corresponding A-scan of the second OCT image (10-2) in the set;

comparing the comparison value with a threshold to determine whether the comparison value is equal to or greater than the threshold;

in case the comparison value is determined to be equal to or greater than the threshold, retaining the respective indication of tissue velocity (v) at the position along the axial direction (z); and

in case the comparison value is determined not to be equal to or greater than the threshold, discarding the respective indication of tissue velocity (v) at the position along the axial direction (z).

9. A data processing apparatus (100) arranged to process each set of optical coherence tomography, OCT, images comprising a first OCT image (10-1) and a second OCT image (10-2) of a plurality of different sets of OCT images in a sequence of OCT images (10) of a common portion of a retina of an eye (20) acquired by a Fourier-domain OCT imaging system (30) to generate a respective indication of a tissue velocity (v) at a position along an axial direction (z) in the OCT images (10), the data processing apparatus (100) being arranged to perform, for each set of the sets of OCT images, processes of:

calculating a respective comparison value being one of:

a value of an image quality metric calculated based on at least one of the first OCT image (10-1) in the set and the second OCT image (10-2) in the set; and

a value of an image similarity metric that provides a measure of a degree of similarity between images, the value of the image similarity metric being calculated based on the first OCT image (10-1) in the set and the second OCT image (10-2) in the set;

comparing the comparison value with a threshold to determine whether the comparison value is equal to or greater than the threshold;

in a case the comparison value is determined to be equal to or greater than the threshold, using a phase value at the position along the axial direction (z) in an A-scan of the first OCT image (10-1) in the set, and a phase value at the position along the axial direction (z) in a corresponding A-scan of the second OCT image (10-2) in the set, in a calculation of the respective indication of tissue velocity (v) at the position along the axial direction (z); and

in case the comparison value is determined not to be equal to or greater than the threshold, omitting a phase value at the position along the axial direction (z) in an A-scan of the first OCT image (10-1) in the set, and a phase value at the position along the axial direction (z) in a corresponding A-scan of the second OCT image (10-2) in the set, in the calculation of the respective indication of tissue velocity (v) at the position along the axial direction (z).

10. The data processing apparatus (100) according to Claim 8 or Claim 9, wherein the comparison value is a maximum value of a calculated cross-correlation between the first OCT image (10-1) and the second OCT image (10-2).

11. The data processing apparatus (100) according to any of Claims 8 to 10, wherein the data processing apparatus (100) is further arranged to:

generate a concatenation (300) of the generated indications of tissue velocity (v) such that the concatenation (300) is indicative of how the tissue velocity (v) at the position along the axial direction (z) changes over time; and integrate the concatenation (300) to generate data indicating an optical path length variation over time at the position along the axial direction.

12. The data processing apparatus (100) according to Claim 11, wherein

in the case where the comparison value is determined not to be equal to or greater than the threshold, the data processing apparatus (100) is arranged to set the respective indication of tissue velocity (v) at the position along the axial direction (z) to indicate zero velocity,
the generated data comprises one or more sets of equal consecutive values, and
the data processing apparatus (100) is further arranged to smooth the generated data by replacing one or more values in a set of the one or more sets of equal consecutive values with one or more estimated values calculated based on neighbouring values that neighbour the set of equal consecutive values in the generated data.

13. The data processing apparatus (100) according to any of Claims 8 to 12, wherein the data processing apparatus (100) is further arranged to process the phase value at the position along the axial direction (z) in the A-scan of the first OCT image (10-1) in the set, and the phase value at the position along the axial direction (z) in the corresponding A-scan of the second OCT image (10-2) in the set, before the calculation of the respective indication of tissue velocity (v) at the position along the axial direction (z), to compensate for a bulk motion of the common portion of the retina during acquisition of the sequence of OCT images (10) by the Fourier-domain OCT imaging system (30).

14. A Fourier-domain optical coherence tomography imaging system (30) comprising the data processing apparatus (100) according to any of Claims 8 to 13.

Fig. 1

230

Working
Memory

220

Processor

I/F

200

210

240

Instruction Store

Computer Program

245

250

260

Fig. 2

Calculate a comparison value for the first OCT image and the second OCT image — S10

Calculate an indication of tissue velocity at a position along the axial direction using a phase value at the position along the axial direction in an A-scan of the first OCT image, and a phase value at the position along the axial direction in a corresponding A-scan of the second OCT image — S20

Compare the comparison value with a threshold to determine whether the comparison value is equal to or greater than the threshold — S30

S40
Is the comparison value equal to or greater than the threshold?

Yes

No

Retain the calculated indication of tissue velocity at the position along the axial direction — S50

S60
Discard the calculated indication of tissue velocity at the position along the axial direction

Fig. 3

Fig. 4A

Fig. 4B

Fig. 5A

Fig. 5B

Fig. 6A

Fig. 6B

Fig. 7

Fig. 8

Fig. 9

Fig. 10

Fig. 11

Calculate a comparison value for the first OCT image and the second OCT image ⟋ S110

Compare the comparison value with a threshold to determine whether the comparison value is equal to or greater than the threshold ⟋ S120

⟋ S130
Is the comparison value equal to or greater than the threshold?

Yes

No

S150

Use a phase value at the position along the axial direction in an A-scan of the first OCT image, and a phase value at the position along the axial direction in a corresponding A-scan of the second OCT, image in the calculation of an indication of tissue velocity at a position along the axial direction ⟋ S140

Omit a phase value at the position along the axial direction in an A-scan of the first OCT image, and a phase value at the position along the axial direction in a corresponding A-scan of the second OCT image, in the calculation of an indication of tissue velocity at a position along the axial direction

Fig. 12

Europäisches
Patentamt
European
Patent Office
Office européen
des brevets

# EUROPEAN SEARCH REPORT

**Application Number**

EP 24 16 2977

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | VIENOLA KARI V. ET AL: "Velocity-based optoretinography for clinical applications", OPTICA, vol. 9, no. 10, 22 September 2022 (2022-09-22), pages 1100-1108, XP093113345, US ISSN: 2334-2536, DOI: 10.1364/OPTICA.460835 Retrieved from the Internet: URL:https://opg.optica.org/directpdfaccess /7483a1bf-9d73-4c2b-ad131ee08afaac42_50633 9/optica-9-10-1100.pdf?da=1&id=506339&seq= 0&mobile=no> * abstract * * Sections 2.D, 4 * * figures 2, 3, * | 1-14 | INV. G06T7/00 G06T7/20 ADD. A61B3/10 A61B3/12 |
| X | MAKITA S ET AL: "Optical coherence angiography", OPTICS EXPRESS,, vol. 14, no. 17, 21 August 2006 (2006-08-21), pages 7821-7840, XP007903893, DOI: 10.1364/OE.14.007821 * Section 2.2.2 * | 1-14 | TECHNICAL FIELDS SEARCHED (IPC) G06T A61B |

The present search report has been drawn up for all claims

| Place of search | Date of completion of the search | Examiner |
|---|---|---|
| Munich | 6 September 2024 | Gitzenis, Savvas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KARI V. VIENOLA et al.** Velocity-based optoretinography for clinical applications. *Optica*, 2022, vol. 9, 1100-1108